# EUROPEAN PATENT APPLICATION

(11) **EP 1 982 693 A1**
(43) Date of publication of application: **22.10.2008**
(21) Application number: 07007890.2
(22) Date of filing: 18.04.2007
(51) Int. Cl.: A61K 8/41, A61K 8/81, A61Q 19/10, A61K 8/97

(54) **Skin detergent**

(71) Applicant: Sansho Cosme Inc., Sumiyosi-ku Osaka-shi Osaka (JP)
(72) Inventor: Hatanaka, Katsuhito, Osaka-shi, Osaka (JP); So, Yukio, Osaka-shi, Osaka (JP); Kozima, Yukari, Osaka-shi, Osaka (JP); Koizumi, Maja, Osaka-shi, Osaka (JP); Ohira, Hitomi, Osaka-shi, Osaka (JP)
(74) Representative: Schildberg, Peter

(57) **Abstract**

A detergent composition for cleaning the skin comprising a mixture of a cream and a powder, which coagulates upon application to the skin and is then rubbed off removing dirt and dead skin with it. The composition comprises water, a polymer having a group which reacts with a cation, and a cationic surface active agent.

## Description

### BACKGROUND OF THE INVENTION

### Field of the Invention

The present invention relates to skin detergent.

### Prior Art

Skin detergent is provided for removing dirt of skin and old or stale keratin, etc. Skin detergent referred to here is the type that is applied on skin and rubbed lightly to become set (like waste of an eraser when used), so that dirt of skin and stale keratin, etc are removed by or together with the skin detergent as set.

Skin detergent hitherto available comprises a mixture of cream and powder, and coagulates and sets due to evaporation of water content to become an eraser's waste-like object, so that the eraser's waste-like object embraces the dirt and sets, thereby showing an effect of cleaning.

But, this type of skin detergent has such defect that it causes users to feel mere setting of cream and does not enable users to readily fully feel the effect of cleaning.

### BRIEF DESCRIPTION OF THE INVENTION

The present invention is skin detergent which is applied on skin and rubbed lightly to become an eraser's waste-like matter, so that dirt of skin, stale or old keratin, or the like are removed together with the eraser's waste-like matter, the skin detergent comprising at least water, polymer having a reactive group to react with cation, and cationic surface active agent.

### DETAILED DESCRIPTION OF THE INVENTION

The present invention relates to skin detergent which is a mixture of respective constituents defined in claim 1.

Polymer having a reactive group to react with cation referred to here is anionic water-soluble polymer, particularly, carboxyvinyl polymer which is produced by polymerization of acrylic monomer and has variations. Usable for the present invention are any which react with cationic surface active agent and become an eraser's waste-like matter (gel), when rubbed.

Content of this polymer in the skin detergent according to the present invention may be preferably 0.1 - 7 wt%(weight%). Insufficient production of gel is at less than 0.1 wt%, and high viscosity of gel and difficulty in use at more than 7 wt%.

Cationic surface active agent may employ any that react with the foregoing polymer, ordinarily, ammonium salt, such as monoalkyltrimethylammonium salt, dialkyltrimethylammonium salt, alkylpyridinium salt, or, dialkyl morihonium salt.

Content of or quantity of mixing cationic surface active agent may be preferably 0.1 to 7 wt% similarly to the above-said polymer.

Polyvalent alcohol may be added to those essential constituents. Polyvalent alcohol may use glycerin, ethylene glycol, polyethylene glycol, or the like. The content or quantity of mixing is not limited but may be preferably 0.5 to 20 wt%.

Next, explanation will be given on Claim 2. Claim 2 relates to skin detergent in which at least one kind of "non-cationic" (i. e., any other than "cationic") surface active agent is also mixed in addition to cationic surface active agent defined in Claim 1. Cationic surface active agent is enough, by itself only, to serve as surface active agent for setting the polymer. For the purpose of providing various effects in addition to that, non-cationic surface active agent is also mixed. This feature is another characteristic of the present invention.

Patters of mixing also other surface active agents as above said are various and mainly those referred to in Claims 3, 4 and 5. Each of them will be explained hereunder. Any of the following three item-groups 1 through 3 is mixed with the essential constituents defined in Claim 1 (any material (s) other than the essential constituents may be also contained).
1 Oil, and nonionic surface active agent
2 Nonionic lipophilic surface active agent, and nonionic hydrophilic surface active agent
3 Amphoteric surfactant

First, the feature mixing oil and nonionic surface active agent in the item-group 1 (the invention set forth in Claim 3) will be detailed.

The purpose of mixing oil referred to here is to provide a feel of being moist without causing skin to get dry and rough after cleaning. Preferable oil usable here may be hydrocarbon, such as squalane, liquid paraffin, and, jojoba oil, and ester, such as isopropyl myristate, ethyl isostearate, and stearyl 2-ethyl hexanoate. Content of or quantity of mixing oil is preferably 0.5 - 10 wt%. Less than 0.5 wt% has almost no effects, and more than 10 wt% gives a feel of being sticky and makes hard to mix.

Nonionic surface active agent is provided for causing water to emulsify oil and is a kind of emulsifier. Hence, the skin detergent in this Example is readily brought into the state of being emulsified to thereby be quite easily usable. Reason for using nonionic ones is that ionic ones are not applicable since they react with the foregoing essential constituents, namely, the foregoing polymer and cationic surface active agent.

Preferable nonionic surface active agent is non-ion type, hydrophilic, and 10 - 18 in HLB (hydrophilic-lipophilic balance) values. Less than 10 in HLB shows almost no effect as the same as of the case not adding nonionic surface active agent. Nonionic surface active agent may be sorbitan oleate, glycerin stearate, etc. Preferable content of or quantity of mixing nonionic surface active agent is 0.1 to 5 wt%, and no effects at less than 0.1wt% and no addition required at more than 5 wt%.

An advantage of this skin detergent is that it gives a feel of being moist after cleaning thanks to mixing of oil. Besides, the skin detergent is high in water-proof property, so that the eraser's waste-like matter according to the present invention is readily formed even on a wet part of skin.

Next, a feature of mixing nonionic lipophilic surface active agent and nonionic hydrophilic surface active agent (cited in the foregoing item-group 2) with the essential constituents will be referred to (the invention set forth in Claim 4).

Nonionic lipophilic surface active agent applied here is 2 to 8 in HLB values, preferably 3 to 7, and may employ polyoxyethylene (5) polyoxypropyleneglycol (HLB 5.8), polyoxyethylene (3) castor oil (HLB 3.0), and, polyoxyethylene (5) behenyl ether (HLB polyoxy 7.0). Content of this nonionic lipophilic surface active agent is preferably 0.1 to 5 wt%, with almost no effects at less than 0.1 wt%, and a feel of being sticky and hardness to mix at more than 5 wt%.

Mixing of nonionic hydrophilic surface active agent may be preferably carried out at 12 to 18 in HLB values, particularly, 13 to 17. Nonionic hydrophilic surface active agent may employ polyoxyethylene (12) secondary alkyl ether (HLB 14.5), polyoxyethylene (9) secondary alkyl ether (HLB 13.5), and polyoxyethylene sorbitan monococoate (20EO) (HLB 16.9) . Content of nonionic hydrophilic surface active agent is preferably 0.1 to 5 wt%, with almost no effects at less than 0.1 wt%, and hardness to mix at more than 5 wt%.

The main point of this feature is that both of lipophilic and hydrophilic nonionic surface active agents are applied. By this, the whole of skin detergent are made into the state of solution (also including a gel state) but not emulsion.

An advantage of the skin detergent in this Example is that it gives a feel of being moist after cleaning thanks to mixing of nonionic lipophilic surface active agent. Besides, the skin detergent is high in water-proof property, so that the skin detergent is usable even on a wet part of skin. And the skin detergent in the state of solution (also including the gel state) has an affinity for skin.

Next, the feature mixing amphoteric surfactant in the item-group 3 (the invention set forth in Claim 5) will be detailed.

Amphoteric surfactant made use of here is a surface active agent having in molecules a positive ion active group and a negative ion active group. Amphoteric surfactant when dissolved in water shows in alkaline range a quality of anionic surface active agent, and in acidic range that of cationic surface active agent. Amphoteric surfactant is, for example, alkyl amino fatty acid salt, alkyl betaine, or, alkyl amine oxide, and may employ lauryl dimethylaminoacetic acid betaine, 2-alkyl-N-carboxymethyl-N-hydroxyethylimidazoliniumbetaine, cocoalkyldimethyl amine oxide solution, sodium N-lauroyl-N'-carboxymethyl-N'-hydroxyethyl ethylenediamine, and, lauroyl amide propyl hydroxy sulfobetaine solution. Those are applicable, while alkyl betaine is preferable since it less stimulates skin. Content of or quantity of mixing amphoteric surfactant is preferably 0.1 to 5 wt%, and more preferably 0.5 to 3 wt%, with a lower effect at less than 0.1 wt%, and much foaming and poor reacting efficiency at more than 5 wt%. Addition of amphoteric surfactant enables the eraser's waste-like matter to be well formed even when skin is wet more or less, and facilitates waterproof property, whereby providing a merit that the skin detergent is usable during user is taking a bath. Amphoteric surfactant less stimulates skin and is higher in safety in comparison with cationic active agent. Moreover, may be added to this feature is a hydrophilic thickener or thickening agent, such as pectin, carageenan, xanthan gum, guar gum, methyl cellulose, etc. Mixing this hydrophilic thickener improves stability of the skin detergent itself and also provides skin with an effect of keeping moisture and enables the eraser' waste-like matter to be readily formed. Content of or quantity of mixing this hydrophilic thickener is not limited but may be preferably around 0.01 to 5 wt%.

Charcoal powder may be added to the skin detergent in those various features (Examples).

Charcoal powder is just the powder of charcoal and may be produced with any methods freely taken. Charcoal powder may be subjected to processing in various manners. Charcoal powder does not mix well with water and is not able to be much mixed. Hence, content of or quantity of mixing charcoal powder is not limited but may be ordinarily around 0.01 to 3 wt%. In place of or in addition to this charcoal powder, there may be applied finely divided charcoal powder folded inside porous silica. The finely divided charcoal powder folded inside porous silica may be provided in such manner that finely divided charcoal powder is coated with silica. Size of finely divided charcoal powder is around several hundreds nm to several hundreds µm (in average granular diameter of mixed matters). Materials for charcoal itself may be ordinary wood, bamboo, or others.

Method of causing finely divided charcoal powder to be folded inside silica is not limited and may be performed, for example, in such manner that finely divided powder of charcoal is mixed with finely divided powder of silica in the same size as of the finely divided power of charcoal. Preferable mixing ratio is charcoal/silica = around 50- 1 in weight ratio. Separately from the above mixture, dispersion medium is prepared by dissolving water-soluble coupling agent (alcoxititanate, etc) in pure water. The fine particles and the dispersion medium are mixed homogeneously so as to prevent secondary coagulating particles from being formed.

Into the mixture of the fine particles and dispersion medium, an organic solvent containing mixed surface active agent is put to obtain emulsion.

The emulsion is subjected to washing precipitates and drying, thereby obtaining "capsulated" charcoal.

Since charcoal folded inside porous silica in this manner is covered with silica (but not wholly), there exist a lot of hydroxyl groups (-OH) around charcoal. By this, charcoal which is hydrophobic is emulsified uniformly with water. In addition, since charcoal is not wholly covered (due to covering with "porous" silica), effects possessed by charcoal are also shown in the invention. Methods for allowing charcoal to be folded by porous silica may be freely taken.

Preferable content of or quantity of mixing charcoal folded inside porous silica is around 0.01 to 5 wt% of the whole, with almost no effect at less than 0.01 wt% and hindrance at more than 5 wt%.

As seen above, mixing fine powder of charcoal enables the skin detergent to be provided with various effects possessed by charcoal itself. In brief, an effect of absorption and that as a scrubbing agent are obtained. An effect of deodorization based on the absorption effect is also able to be expected.

To emphasize further visually the effects of charcoal, other black pigment or dye may be mixed and may employ carbon, squid ink, graphite, black dye, paint, and others. Content of or quantity of mixing the black pigment, dye, or others may be preferably about 0 to 3 wt%.

Water is added to those various constituents to make 100 wt% and finish the invention. As long as there is no deviation from the gist of the present invention, any other materials, such as plant extracts, vitamins, coloring agents, perfume, etc, may be added as a constituent.

A manner of usage of the skin detergent according to the present invention is that the skin detergent is applied on skin (a wet state is not problematic) in a suitable quantity and rubbed lightly as massaging lightly, so that the liquid (the skin detergent) sets to become an eraser's waste-like matter which embraces dirt of skin and stale keratin, etc and removes them from skin.

Thereafter, the eraser's waste-like matter is washed away with water or warm water, which is quite simple and easy activity. And skin care which is hitherto ordinarily to be taken after cleaning with skin detergent may or may not be taken.

### PREFERRED EMBODIMENTS OF THE INVENTION

First, an example in relation to Claim 1 will be referred to.

The following constituents were applied mixed to prepare the Examples and Comparative examples.

Carboxyvinyl polymer (trademark "Carbopol 940", Noveon Company) was used as polymer having a reactive group to react with cation, and behenyl trimethyl ammonium salt as cationic surface active agent, and furthermore, glycerin as polyvalent alcohol.

Those were mixed into the invention in the ratio shown in Table 1, exemplifying as Examples 1 to 3, with the conventional type of skin detergent using cream being exemplified as Comparative example.

Respective examples were applied, for experiment, on skin of 10 subjects in their twenties and lightly massaged over the applied examples to allow the examples to set. Various effects were judged and the result is as described in Table 1. The result is an average taken from the ten subjects.

In Table 1, extent of formation of the eraser's waste-like matter is indicated by the markings ⊚ which means "Quite excellent", O"Good", Δ. "Eraser's waste-like matter appears but in a rather small quantity", and X"Almost not forming the eraser's waste-like matter".

Next, the examples in relation to Claim 3 will be explained.

Regarding the examples, the constituents mixed, their quantities and effects are shown in Table 2. The manner performing the experiment is the same as of Table 1.

The polymer having a reactive group reacting with cation, cationic surface active agent, and polyvalent alcohol employed here are the same as those used in the example shown in Table 1. As higher alcohol, behenyl alcohol was used.

Squalane was employed as oil, and sorbitan monooleate as nonionic surface active agent.

In table 2, water-proof property was judged from that the skin detergent was rubbed lightly with users' hands fully wet or moistened so that eraser's waste-like matter was or was not recognized visually. The marking ⊚ indicates "The eraser's waste-like matter was recognized at once.", and 0 "recognized a little".

For stability, ⊚ indicates "Quite stable", and 0 "Stable".

The examples shown in Table 2 are superior in a feel of emollient effect and a water-proof property as a whole.

Next, examples in relation to Claim 4 will be referred to.

Regarding the examples, the constituents mixed, their quantities and effects are shown in Tables 3 and 4. The manner performing the experiment is the same as of Table 1.

Names of constituents shown in Tables 3 and 4 are as follows.
- A:: Carboxyvinyl polymer (Trademark "Carbopol 940", Noveon Company)
- B:: Behenyl trimethyl ammonium salt
- C:: Polyoxyethylene (5) polyoxypropyleneglycol (HLB 5.8)
- D:: Polyoxyethylene (3) castor oil (HLB 3.0)
- E:: Polyoxyethylene (5) behenyl ether (HLB 7.0)
- F:: Polyoxyethylene (12) secondary alkyl ether (HLB 14.5)
- G:: Polyoxyethylene (9) secondary alkyl ether (HLB 13.5)
- H:: Polyoxyethylene sorbitan monococoate (20EO) (HLB 16.)
- I:: Glycerin
- J:: Water

The effects shown in the Table is based on the same standard for judgment as in Table 2.

The examples are superior as a whole in appearance of the eraser's waste-like matter, water-proof property and others.

Next, examples in relation to Claim 5 will be referred to.

Regarding the examples, the constituents mixed, their quantities and effects are shown in Table 5. The manner performing the experiment is the same as of Table 1.

Names of constituents shown in Table 5 are as follows.
- A:: Carboxyvinyl polymer (Trademark "Carbopol 940", Noveon Company)
- B:: Behenyl trimethyl ammonium salt
- C:: Amphoteric surfactant,
lauryl dimethylaminoacetic acid betaine
- D:: Amphoteric surfactant, 2-alkyl-N-carboxymethyl-N-hydroxyethylimidazoliniumbetaine
- E:: Amphoteric surfactant,
cocoalkyldimethyl amine oxide solution
- F:: Thickening agent, pectin
- G:: Glycerin
- H:: Water

The effects shown in the Table is based on the same standard for judgment as in Table 2.

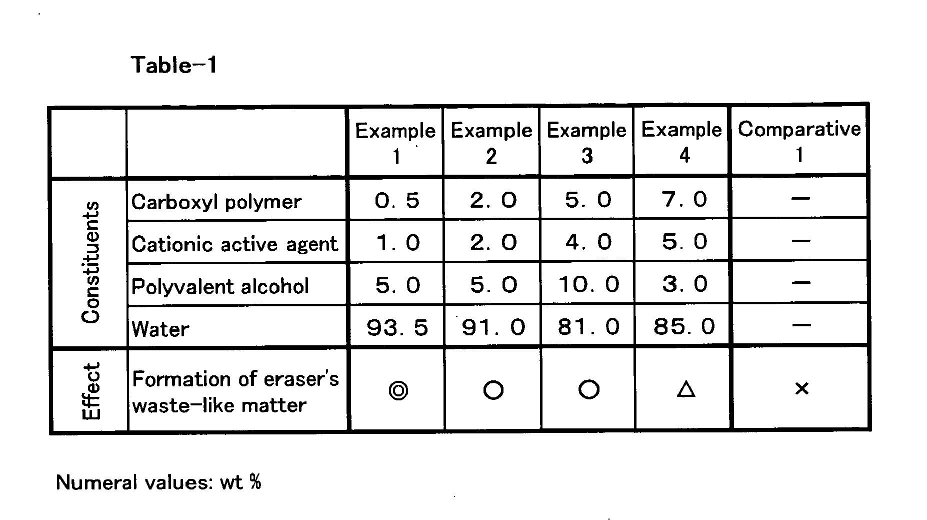

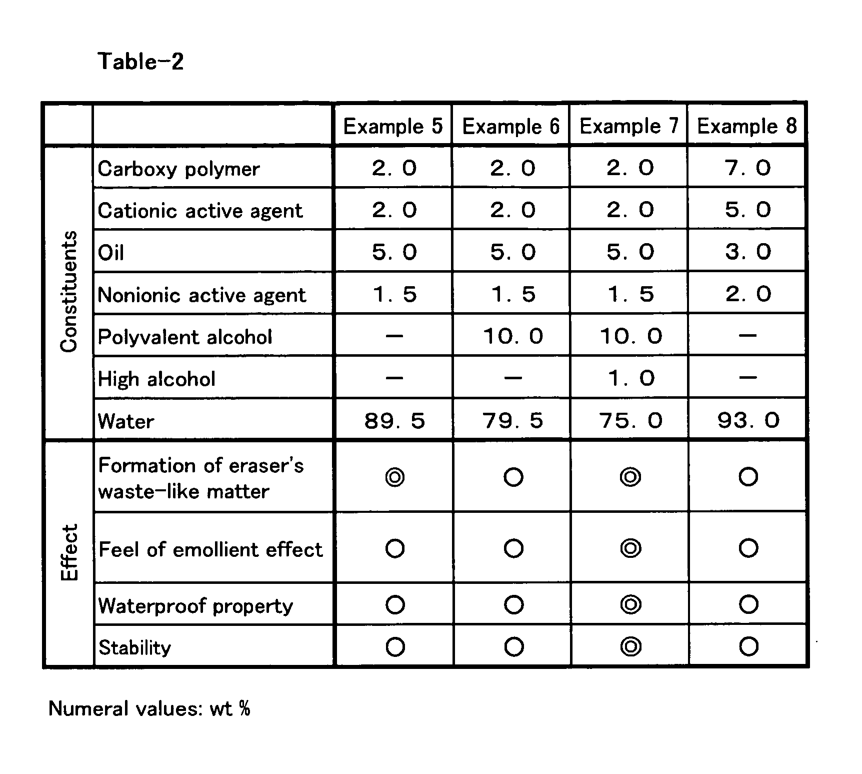

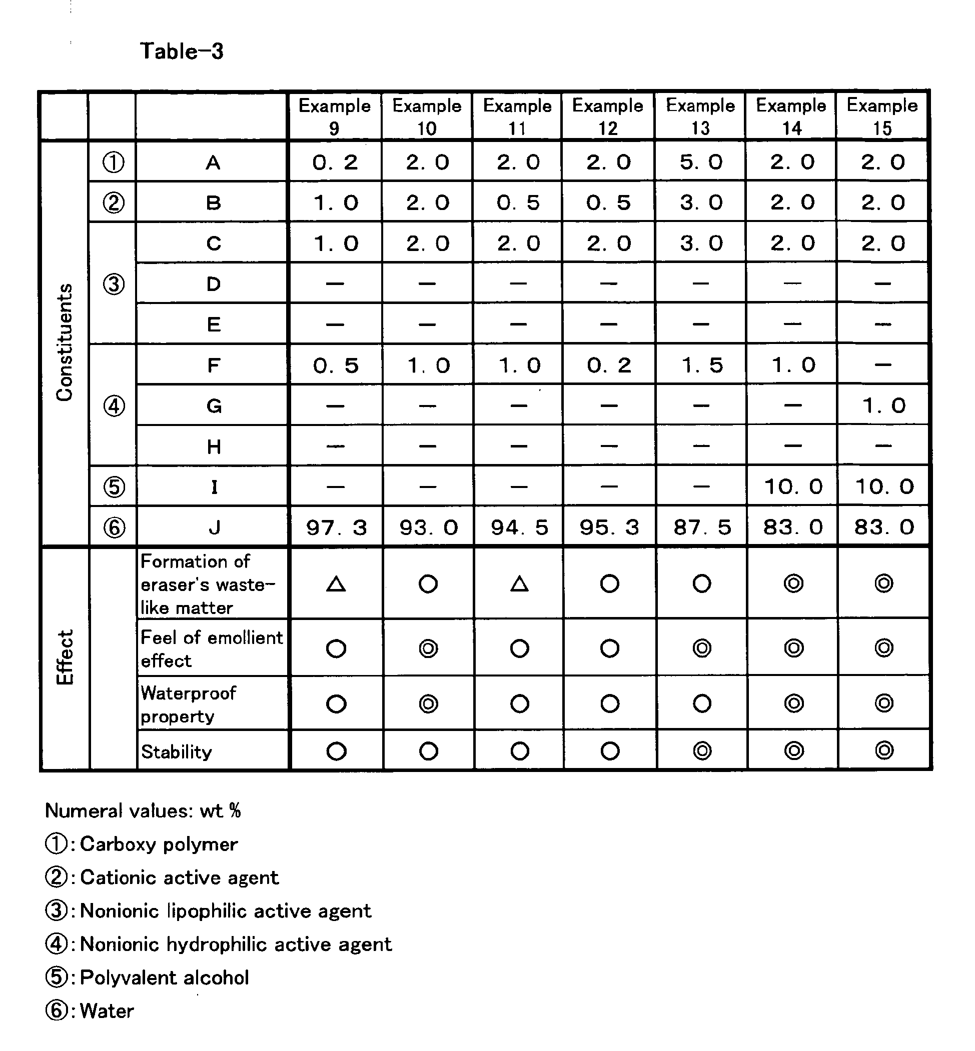

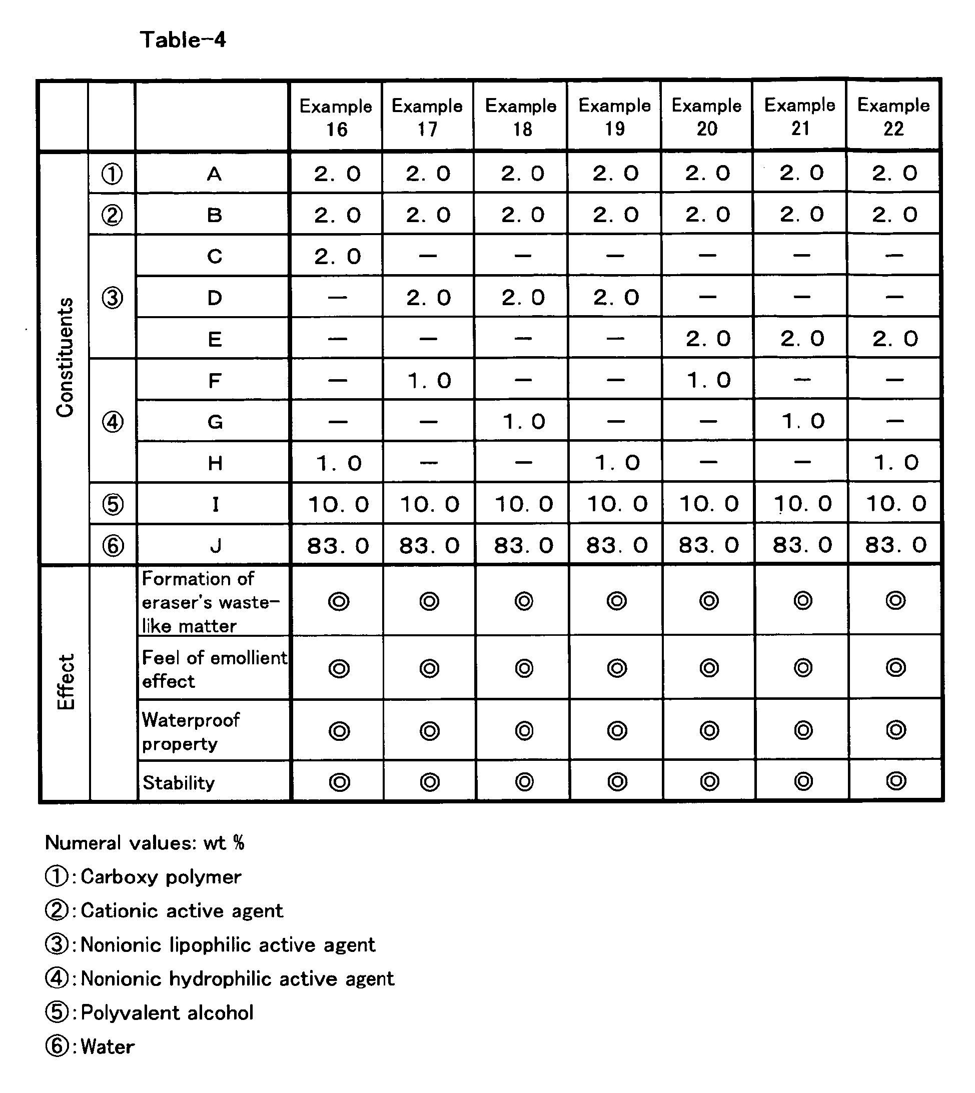

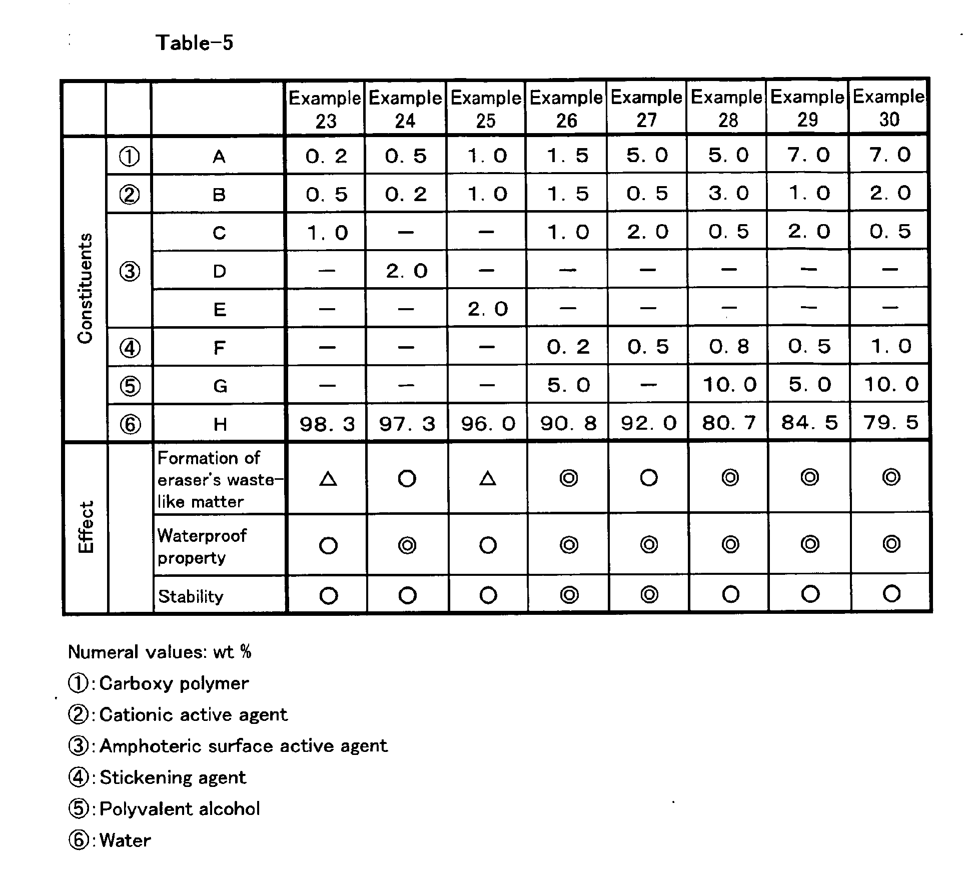

## Claims

1. Skin detergent which is applied on skin and rubbed to become an eraser's waste-like matter, so that dirt of skin, stale or old keratin, or the like are removed together with the eraser's waste-like matter, the skin detergent comprising at least water, polymer having a reactive group to react with cation, and cationic surface active agent.

2. Skin detergent as set forth in Claim 1 wherein the skin detergent contains at least one kind of non-cationic surface active agent, in addition to cationic surface active agent.

3. Skin detergent as set forth in Claim 2 wherein the skin detergent contains oil and nonionic surface active agent.

4. Skin detergent as set forth in Claim 2 wherein the skin detergent contains nonionic lipophilic surface active agent and nonionic hydrophilic surface active agent.

5. Skin detergent as set forth in Claim 2 wherein the skin detergent contains amphoteric surfactant.

6. Skin detergent as set forth in Claims 1 through 5 wherein the skin detergent contains charcoal powder.
